# EUROPEAN PATENT APPLICATION

(11) **EP 4 253 556 A1**
(43) Date of publication of application: **04.10.2023**
(21) Application number: 21897938.3
(22) Date of filing: 24.11.2021
(51) Int. Cl.: C12Q 1/25, C12N 5/071

(54) **METHOD FOR EVALUATING EXCRETION OF SUBSTANCE OF INTEREST BY HUMAN HEPATOCYTE-LIKE CELL**

(30) Priority: 25.11.2020 JP 2020195476
(71) Applicant: JSR Corporation, Tokyo 105-8640 (JP); Kendai Translational Research Center, Takasaki-shi, Gunma 370-0033 (JP)
(72) Inventor: MASUDA, Norio, Tokyo 105-8640 (JP); OGIHARA, Takuo, Takasaki-shi, Gunma 370-0033 (JP)
(74) Representative: TBK
(86) International application number: PCT/JP2021/042883
(87) International publication number: WO 2022/113965

(57) **Abstract**

A method is provided for evaluating excretion of a substance of interest by a human hepatocyte-like cell, the method including: preparing a sac-like-material-containing solution that contains a sac-like material which is produced in vitro and has a membrane of a human hepatocyte-like cell and 0% to 10% by volume of a suspended extracellular matrix; placing the substance of interest in the sac-like-material-containing solution to bring the substance of interest into contact with the sac-like material; and extracting the sac-like material from the sac-like-material-containing solution and measuring a concentration of the substance of interest or a metabolite thereof excreted into an inner cavity of the sac-like material.

## Description

### [Technical Field]

The present invention relates to a method for evaluating excretion of a substance of interest by a human hepatocyte-like cell and a method for producing a sac-like material having a membrane of a human hepatocyte-like cell.

Priority is claimed on Japanese Patent Application No. 2020-195476, filed November 25, 2020, the content of which is incorporated herein by reference.

### [Background Art]

The liver is composed of hepatic parenchymal cells, which are responsible for essential liver functions, and a group of hepatic nonparenchymal cells that support the proliferation and survival of the hepatic parenchymal cells. The hepatic parenchymal cells are also referred to as hepatocytes. The group of hepatic nonparenchymal cells is composed of hepatic stellate cells, sinusoidal endothelial cells, Kupffer cells, bile duct epithelial cells, and the like.

The hepatocytes convert exogenous drugs and substances generated due to metabolism into substances having low toxicity, which are excreted through the following two excretion pathways. The first is a pathway in which the converted substances having low toxicity are excreted into the bile canaliculus together with the bile and excreted out of the body as feces by passing through the gall bladder and the intestine. In the second pathway, the converted substances having low toxicity are excreted into the sinusoidal blood vessels, reach the kidney, and are then excreted out of the body as urine. In these two excretion pathways, it is known that both transporters that incorporate exogenous drugs and substances generated during metabolism into the hepatocytes and transporters that excrete converted substances having low toxicity are different.

In drug discovery, a large number of candidate drugs identified in initial screening are eliminated at the preclinical test stage. In addition, the fact that a number of candidate drugs have been eliminated leads to the loss of medical treatment opportunities for patients. By the way, the preclinical test is composed of an efficacy screening of candidate drugs and a safety test of candidate drugs, and it is known that in vitro preclinical models are useful for these screenings (for example, see Non-Patent Document 1 to Non-Patent Document 3).

### [Citation List]

### [Non-Patent Documents]

[Non-Patent Document 1]
   Oshikata MA et al., "Development of an oxygenation culture method for activating the liver-specific functions of HepG2 cells utilizing a collagen vitrigel membrane chamber.", Cytotechnology, Vol. 68, pp. 1801-1811, 2016.
[Non-Patent Document 2]
   Jang KJ et al., "Reproducing human and cross-species drug toxicities using a Liver-Chip", Sci. Transl. Med., Vol. 11, Issue 517, eaax5516, 2019, doi: 10.1126/scitranslmed.aax5516.
[Non-Patent Document 3]
   Swift B et al., "Sandwich-Cultured Hepatocytes: An In Vitro Model to Evaluate Hepatobiliary Transporter-Based Drug Interactions and Hepatotoxicity", Vol. 42, Issue 3, pp. 446-471, 2010.

### [Summary of Invention]

### [Technical Problem]

The present invention has been made in consideration of the above circumstances and provides a method for evaluating excretion of a substance of interest by a human hepatocyte-like cell using an evaluation model for evaluating excretion by a human hepatocyte-like cell, and a method for producing the evaluation model.

### [Solution to Problem]

That is, the present invention includes the following aspects.
(1) A method for evaluating excretion of a substance of interest by a human hepatocyte-like cell, the method including:
   preparing a sac-like-material-containing solution that contains a sac-like material which is produced in vitro and has a membrane of a human hepatocyte-like cell and 0% to 10% by volume of a suspended extracellular matrix;
   placing the substance of interest in the sac-like-material-containing solution to bring the substance of interest into contact with the sac-like material; and
   extracting the sac-like material from the sac-like-material-containing solution and measuring a concentration of the substance of interest or a metabolite thereof excreted into an inner cavity of the sac-like material.
(2) The method according to (1), in which the human hepatocyte-like cell is a cell capable of being subjected to organoid culture.
(3) The method according to (1) or (2), in which the human hepatocyte-like cell has at least one transporter selected from the group consisting of BSEP, MRP2, P-gp, MATE, and BCRP.
(4) The method according to any one of (1) to (3), in which in step 3, the concentration of the substance of interest or the metabolite thereof excreted into the inner cavity of the sac-like material is measured after degrading the sac-like material.
(5) The method according to any one of (1) to (4), in which in step 3, the concentration of the substance of interest or the metabolite thereof excreted into the inner cavity of the sac-like material is measured by liquid chromatography-mass spectrometry.
(6) The method according to any one of (1) to (5), in which the inner cavity of the sac-like material contains bile.
(7) The method according to any one of (1) to (6), in which the membrane of the human hepatocyte-like cell is a single layer membrane.
(8) A method for producing a sac-like material having a membrane of a human hepatocyte-like cell, the method including:
   embedding and culturing a human hepatic progenitor cell or a human liver cell in an extracellular matrix to form a culture;
   dispersing the culture to form a dispersion; and
   subjecting the dispersion to suspension culture in a culture medium, in which 0% to 10% by volume of an extracellular matrix is suspended with respect to a total volume of the culture medium, to form the sac-like material.

### [Advantageous Effects of Invention]

According to the method of the above aspect, it is possible to provide a method for evaluating excretion of a substance of interest by a human hepatocyte-like cell using an evaluation model for evaluating excretion by a human hepatocyte-like cell. According to the production method of the above aspect, it is possible to provide an evaluation model for evaluating excretion by a human hepatocyte-like cell.

### [Brief Description of Drawings]

FIG. 1 shows a bright-field image of an evaluation model in Experimental Example 1.
FIG. 2 shows images of an evaluation model in which Rho123 is incorporated into a sac-like material in Experimental Example 1. (a) is a bright-field image, and (b) is a fluorescence image.
FIG. 3 shows images of a culture into which Rho123 is incorporated into the inner cavity of a culture in Experimental Example 5. (a) is a bright-field image, and (b) is a fluorescence image.
FIG. 4 shows an optical microscopic image of a sac-like material.
FIG. 5 shows images of a culture into which Rho 123 is incorporated into the inner cavity of a culture in Experimental Example 6.

### [Description of Embodiments]

Hereinafter, the present invention will be described in more detail with reference to embodiments; however, the present invention is not limited to the following embodiments.

Unless otherwise specified, each component as an exemplary example in the present specification, for example, one kind of component contained in a culture medium or one kind of component used in each step can be used alone, or two or more kinds thereof can be used in combination.

In the present specification, a notation representing a numerical range as "A to B" has the same meaning as "A or more and B or less". In addition, in the present specification, a notation representing a numerical range as "A to B, preferably a to b" has the same meaning as "A or more and B or less", "A or more and b or less", "a or more and B or less", and "a or more and b or less".

In the present specification, the terms "culture medium including substance X" and "in the presence of substance X" mean a culture medium to which an exogenous substance X has been added, a culture medium including an exogenous substance X, or in the presence of an exogenous substance X. That is, in the case where a cell or tissue present in the culture medium expresses, secretes, or produces the substance X endogenously, the endogenous substance X is distinguished from the exogenous substance X, and it is to be noted that a culture medium that does not include the exogenous substance X does not fall under the category of "culture medium including substance X" even in the case where the culture medium includes the endogenous substance X.

### [Method for evaluating excretion of substance of interest by human hepatocyte-like cell]

The method according to the present embodiment is a method for evaluating excretion of a substance of interest (hereinafter, also referred to as a "substrate") by a human hepatocyte-like cell (hereinafter, also referred to as "the evaluation method according to the present embodiment"), where it has the following step 1 to step 3:
preparing a sac-like-material-containing solution that contains a sac-like material (hereinafter, also referred to as "the present evaluation model") which is produced in vitro and has a membrane of a human hepatocyte-like cell and 0% to 10% by volume of a suspended extracellular matrix (hereinafter, also referred to as "step 1");
placing the substance of interest in the sac-like-material-containing solution to bring the substance of interest into contact with the sac-like material (hereinafter, also referred to as "step 2"); and
extracting the sac-like material from the sac-like-material-containing solution and measuring a concentration of the substance of interest or a metabolite thereof excreted into the inside of the sac-like material (hereinafter, also referred to as "step 3").

The present evaluation model is suitably used as a biomimetic model (Micro Physiological System, MPS) for evaluating excretion of a substance of interest by a human hepatocyte cell. In the biomimetic model, in order to mimic the structure of human liver tissue in vivo, it is necessary to impart aligning properties to human hepatocytes to be arranged in a membranous shape. In the related art, human hepatic progenitor cells of human liver cells have been cultured in a state of being embedded in an extracellular matrix in order to impart aligning properties to human hepatocytes to be arranged in a membranous shape. The sac-like material obtained in this way is in a state of being embedded in the extracellular matrix.

In order to quantify the concentration of the substance of interest incorporated into the sac-like material by excretion, it is necessary to remove the extracellular matrix physically or chemically using an enzyme or the like in the case of extracting the substance of interest incorporated into the sac-like material. However, in the case where the extracellular matrix is removed, there is a risk that the human hepatocyte-like cell membrane of the sac-like material is broken and the substance of interest inside the sac-like material leaks out. On the other hand, in the case where the substance of interest is extracted from the sac-like material without removing the extracellular matrix and then the concentration of the substance of interest is quantified by LC-MS or the like, the extracellular matrix becomes noise, and thus the amount of the substance of interest cannot be measured accurately. For the above reasons, in the sac-like material in a state of being embedded in the extracellular matrix in the related art, it has been difficult to quantify the substance of interest excreted into the sac-like material.

In contrast, in the evaluation method according to the present embodiment, although 0% to 10% by volume of an extracellular matrix is present in the sac-like-material-containing solution, the sac-like material is not embedded in the extracellular matrix. Therefore, the sac-like material can be extracted from the sac-like-material-containing solution without breaking the sac-like material, and the concentration of the substance of interest incorporated into the sac-like material can be easily quantified.

According to the evaluation method of the present embodiment, the metabolism of a substance of interest, the drug interaction, the hepatotoxicity, the transporter activity, and the like by the human hepatocyte-like cell can be subjected to highly accurate quantitative evaluation by liquid chromatography-mass spectrometry instead of the low accuracy evaluation in the related art, which has been carried out based on the fluorescence intensity using a substrate having a fluorescence label substrate or bile.

### <Step 1>

In step 1, a sac-like-material-containing solution, which contains the present evaluation model produced in vitro and 0% to 10% by volume of a suspended extracellular matrix, is prepared.

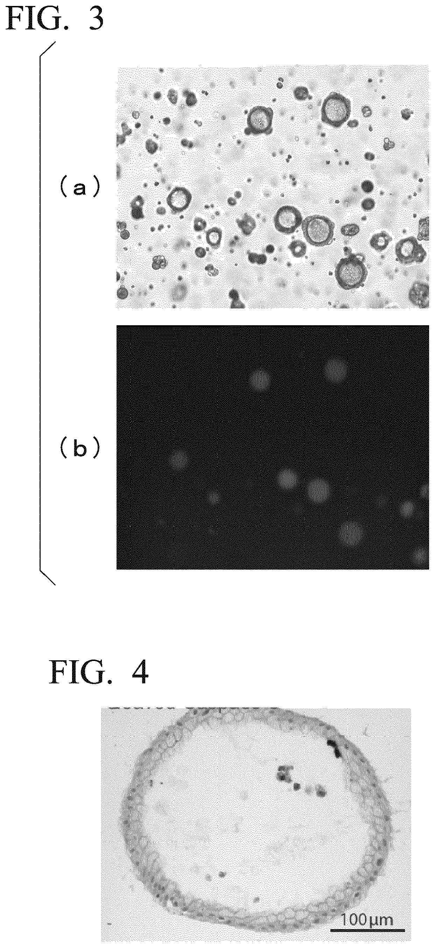

The sac-like material as the present evaluation model is also referred to as a cyst and has a sack-like structure formed from a membrane of a human hepatocyte-like cell, as shown in FIG. 4.

In the present evaluation model, it is preferable that a bile canaliculus-like structure be constructed between human hepatocyte-like cells. In the case of having a bile canaliculus-like structure between human hepatocyte-like cells, it is possible to have a tight junction.

The inner cavity of the present evaluation model preferably contains bile. Further, the human hepatocyte-like cell of the present evaluation model preferably has at least one transporter selected from the group consisting of bile salt export pump (BSEP), multidrug resistance-associated protein 2 (MRP2), P-glycoprotein (P-gp), multidrug and toxin extrusion (MATE), and breast cancer resistance protein (BCRP). Each of these transporters is known as a bile excretion transporter for a different substance.

In the case in which the present evaluation model has a bile canaliculus-like structure, and the human hepatocyte-like cell of the present evaluation model has the transporter described above, a substance of interest of a metabolite thereof incorporated into the human hepatocyte-like cell can be excreted, by the transporter described above, out of the human hepatocyte-like cell by passing through the bile canaliculus-like structure from the inside of the human hepatocyte-like cell and can be accumulated, together with bile, in the inner cavity of the sac-like material.

In addition to the transporter described above, the human hepatocyte-like cell of the present evaluation model can further have an incorporation transporter such as Na+ - taurocholate cotransporting polypeptide (NTCP), an organic anion transporting polypeptide (OATP) family transporter, or organic cation transporter 1 (OCT1).

The human hepatocyte-like cell of the present evaluation model can further express a gene of metabolic enzymes such as CYP1A2, CYP2A6, CYP2C9, CYP2C19, CYP2D6, CYP2E1, and CYP3A4.

The membrane of the human hepatocyte-like cell in the present evaluation model is preferably a single layer membrane. In the case where the membrane of the human hepatocyte-like cell is a single layer membrane, the effect of a substance of interest per cell can be evaluated by the present evaluation model.

The volume of the present evaluation model is 4e⁻⁶ to 3e⁻² mm³, preferably 3e⁻⁵ to 1e⁻² mm³, and more preferably 1e⁻⁴ to 4e⁻³ mm³.

From the viewpoint of stability of functions and properties, the human hepatocyte-like cell of the present evaluation model is preferably a cell that can be subjected to organoid culture. Organoid culture is a three-dimensional self-organizing culture of stem cells, and cells obtained by organoid culture are also referred to as an organoid.

The proportion of the sac-like material contained in the sac-like-material-containing solution is 10 to 2,000 pieces/mL, preferably 50 to 1,000 pieces/mL, and more preferably 100 to 500 pieces/mL.

Examples of the extracellular matrix include a component contained in the basement membrane and a glycoprotein present in the intercellular space. Examples of the component contained in the basement membrane include type IV collagen, laminin, heparan sulfate proteoglycan, and entactin.

The proportion of the extracellular matrix contained in the sac-like-material-containing solution is 0% to 10% by volume with respect to the total volume of the sac-like-material-containing solution, where it is preferably 0.001% to 5% by volume, more preferably 0.1% to 4% by volume, and still more preferably 1% to 3% by volume.

The sac-like-material-containing solution is preferably a solution containing a culture medium to be used in suspension culture, an extracellular matrix, and a sac-like material having a membrane of a human hepatocyte-like cell in "Method for producing sac-like material having membrane of human hepatocyte-like cell" described later.

### <Step 2>

In step 2, the substance of interest prepared in step 1 is placed in the sac-like-material-containing solution, and the substance of interest is brought into contact with the present evaluation model.

Examples of the substance of interest include a natural compound library, a synthetic compound library, an existing drug library, and a metabolite library. As the substance of interest, organic compounds or inorganic compounds having various molecular sizes can be used. Examples of the organic compound include a nucleic acid, a peptide, a protein, a lipid (a simple lipid or a composite lipid (a phosphoglyceride, a sphingolipid, a glycosyl glyceride, a cerebroside, or the like), a prostaglandin, an isoprenoid, a terpene, a steroid, a polyphenol, catechin, and a vitamin (B1, B2, B3, B5, B6, B7, B9, B12, C, A, D, E), or the like).

Examples of the substance of interest include components contained in pharmaceutical drugs, nutritional food products, and the like. Examples of the substance of interest include a plant extract, a cell extract, and a culture supernatant. In the case of simultaneously adding two or more kinds of substances of interest, it is possible to examine the interaction, synergy, and the like between the substances of interest. The substance of interest may be derived from natural products or may be artificially synthesized.

The period during which the substance of interest is brought into contact with the present evaluation model is generally 10 minutes or more and 3 days or less, preferably 30 minutes or more and 1 day. The contact between the substance of interest and the present evaluation model can be dividedly carried out a plurality of times.

The substance of interest is incorporated by the human hepatocyte-like cell of the present evaluation model, and subsequently, the incorporated substance of interest is excreted into the inner cavity of the present evaluation model, or the incorporated substance of interest is metabolized by metabolic enzymes of the human hepatocyte-like cell of the present evaluation model and excreted into the inner cavity of the present evaluation model.

### <Step 3>

In step 3, the present evaluation model is extracted from the sac-like material-like containing solution after step 2, and the concentration of the substance of interest or the metabolite thereof excreted into the inner cavity of the present evaluation model is measured.

Before measuring the concentration, it is preferable to wash the present evaluation model extracted from the sac-like material-like containing solution using phosphate-buffered saline (PBS) or the like. This can reduce the carry-over of the extracellular matrix into the concentration measurement environment.

It is preferable to extract the substance of interest in the inner cavity of the present evaluation model by degrading the membrane of the human hepatocyte-like cell of the present evaluation model. Examples of the method for degrading the membrane of the human hepatocyte-like cell include a chemical method using methanol or the like and a physical method such as homogenization.

For the measurement of the concentration of the substance of interest or the metabolite thereof, it is possible to employ mass spectrometry, liquid chromatography, an immunological method, or the like, depending on the kind of the substance of interest or the metabolite thereof. Examples of the immunological method include a fluorescence immunoassay method (an FIA method) and an enzyme immunoassay method (an EIA method). Among them, it is preferable to measure the concentration of the substance of interest or the metabolite thereof by a liquid chromatography-mass spectrometry (LC-MS) method due to the excellent quantification.

Step 3 makes it possible to evaluate the toxicity of the substance of interest. For example, the state of the human hepatocyte-like cell after being brought into contact with a substance of interest is examined to evaluate the toxicity of the substance of interest. The evaluation method for a human hepatocyte-like cell can be carried out based on the viability, the cell morphology, or the abundance of liver damage markers (for example, GOT and GPT) in the culture solution.

### [Method for producing sac-like material having membrane of human hepatocyte-like cell]

The method for producing a sac-like material having a membrane of a human hepatocyte-like cell according to the present embodiment (hereinafter, also referred to as "the production method according to the present embodiment") has the following step A to step C:
embedding and culturing a human hepatic progenitor cell or a human liver cell in an extracellular matrix to form a culture (hereinafter, also referred to as "step A");
dispersing the culture to form a dispersion (hereinafter, also referred to as "step B"); and
subjecting the dispersion to suspension culture in a culture medium, in which 0% to 10% by volume of an extracellular matrix is suspended with respect to a total volume of the culture medium, to form the sac-like material having a membrane of the human hepatocyte-like cell (hereinafter, also referred to as "step C").

A sac-like material having a membrane of the human hepatocyte-like cell produced according to the production method according to the present embodiment can be used as the present evaluation model for the evaluation method according to the present embodiment.

Hereinafter, each of the steps of the production method according to the present embodiment will be described in detail.

### <Step A>

In step A, a human hepatic progenitor cell or a human liver cell is embedded and cultured in an extracellular matrix to form a culture. The culture formed contains human hepatocyte-like cells.

In the case of being cultured in a culture medium containing factors selected from TGFβ signaling promoters, for example, Wnt, Nodal, and activin A, and any combination thereof, a human hepatic progenitor cell is induced from a human pluripotent stem cell to differentiate into embryonic endoderm. Next, the culture is carried out in a culture medium containing factors selected from FGF, BMP, and any combination thereof, whereby human hepatic progenitor cells can be obtained. Examples of the human pluripotent stem cell include a human embryonic stem cell (hESC) and a human induced pluripotent stem cell (hiPSC).

Human liver cells generally include hepatic parenchymal cells and hepatic nonparenchymal cells such as ductal cells, sinusoidal endothelial cells, Kupffer cells, mesothelial cells, and hepatic stellate cells. Among these, hepatic nonparenchymal cells are preferably included.

Human liver cells are preferably used in step A since a human hepatocyte-like cell of which the gene phenotype is close to that of the wild-type human hepatocytes can be obtained.

In the case of being embedded and cultured in an extracellular matrix, human hepatic progenitor cells or human liver cells proliferate and mature to form human hepatocyte-like cells. Examples of the extracellular matrix include those of exemplary examples in step 1.

Examples of the method for embedding and culturing a human hepatic progenitor cell or a human liver cell in an extracellular matrix include a method in which human hepatic progenitor cells or human liver cells are mixed with an extracellular matrix precursor, the extracellular matrix precursor is polymerized to form an extracellular matrix, on which a culture medium is subsequently overlaid to carried out culturing.

The culture conditions in step A are generally 30°C or higher and 40°C or lower and preferably 37°C. Other culture conditions are such that, in general, culturing is carried out in an atmosphere under the condition of a CO₂ concentration of about 5% by volume.

The culture time can be appropriately adjusted according to the number of cells, the state of the cells, and the like. The culture medium can be changed every 1 to 3 days. In addition, in step A, a culture having stable functions and properties can be obtained by subculturing a plurality of times.

The culturing in step A is preferably organoid culture. In organoids obtained according to organoid culture, the expression levels of transporters and the expression levels of genes of metabolic enzymes are high, and membranes of human hepatocyte-like cells of the sac-like material have high trans epithelial electrical resistance, and thus a tight junction can be formed.

### (Culture medium)

The culture medium used in step A preferably contains at least one selected from the group consisting of factors related to proliferation such as a mitogenic growth factor, a Wnt signaling promoter, a Rho kinase (ROCK) signaling inhibitor, an IL-6 family cytokine, and nicotinamide; factors that inhibit differentiation such as transforming growth factor β (TGF-β) signaling inhibitor; factors related to maturation such as retinoic acid, a γ-secretase inhibitor (DAPT), dimethylsulfoxide (DMSO), and dexamethasone (Dex); and factors related to activation of cellular receptors such as forskolin.

In the case of culturing human hepatic progenitor cells, the culture medium preferably contains a factor related to maturation and preferably contains at least one factor selected from DAPT, DMSO, and Dex.

In the case of culturing human liver cells, the culture medium preferably contains a factor related to maturation, a factor related to proliferation, a factor inhibiting differentiation, and a factor related to activation of cellular receptors, where it preferably contains DAPT as a factor related to maturation, and an IL-6 family cytokine, a mitogenic growth factor, a Wnt signaling promoter, and a ROCK signaling inhibitor as factors related to proliferation.

### (1) Mitogenic growth factor

The mitogenic growth factor is a factor that induces the initiation of cell division. Examples of the mitogenic growth factor include epidermal growth factor (EGF), fibroblast growth factor (FGF), and hepatocyte growth factor (HGF). The FGF is preferably the one that can bind to FGF receptor 2 (FGFR2) or FGF receptor 4 (FGFR4), where it is preferably FGF2, FGF4, FGF7, or FGF10 and particularly preferably FGF10. Among the above, EGF, FGF10, or HGF is preferable, and it is more preferable to contain all of EGF, FGF10, and HGF.

The concentration of each of EGF and HGF contained in the culture medium is generally 10 ng/mL to 500 ng/mL, preferably 15 ng/mL to 100 ng/mL, more preferably 20 ng/mL to 80 ng/mL, and still more preferably 25 ng/mL and 50 ng/mL.

The concentration of the FGF contained in the culture medium is generally 20 ng/mL to 500 ng/mL, preferably 50 ng/mL to 300 ng/mL, more preferably 80 ng/mL to 150 ng/mL, and still more preferably 90 ng/mL to 110 ng/mL.

### (2) Wnt signaling promoter

The Wnt signal is a signal related to the proliferation of stem cells and the maintenance of differentiative properties. A Wnt signaling promoter can upregulate wnt signaling. Examples of the Wnt signaling promoter include a Wnt agonist, an Lgr5 agonist, a glycogen synthase (GSK) inhibitor.

Examples of the Wnt agonist include Wnt family members such as Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16. Among these, Wnt3a is preferable.

Afamin is known to contribute to the stabilization and solubilization of Wnt family members. A Wnt agonist can be used as a complex of a Wnt family member and Afamin.

The proportion of the Wnt agonist contained in the culture medium is generally 1% to 50% by volume with respect to the total volume of the culture medium, where it is preferably 10% to 30% by volume and more preferably 15% to 25% by volume.

Examples of the Lgr5 agonist include R-spondin 1, R-spondin 2, R-spondin 3, and R-spondin 4. Among these, R-spondin 1 is preferable.

The proportion of the Lgr5 agonist contained in the culture medium is generally 1% to 50% by volume with respect to the total volume of the culture medium, where it is preferably 5% to 30% by volume and more preferably 5% to 15% by volume.

Examples of the GSK inhibitors include CHIR99021 (CAS Number: 252917-06-9), SB216763 (CAS Number: 280744-09-4), SB415286 (CAS Number: 264218-23-7), CHIR98014 (CAS Number: 252935-94-7), AZD1080 (CAS number: 612487-72-6), and LY2090314 (CAS number: 603288-22-8). Among these, CH1R99021 is preferable.

The concentration of the GSK inhibitor contained in the culture medium is generally 0.1 µM to 10 µM. It is to be noted that, here, "M" means mol/L, and the same applies hereinafter.

As the Wnt signaling promoter, it is preferable to use a combination of a Wnt agonist and an Lgt5 agonist.

### (3) ROCK signaling inhibitor

The downregulation of ROCK signaling makes it possible for hepatocytes to acquire proliferation ability. Examples of the ROCK signaling inhibitor include Y-27632 (CAS number: 146986-50-7), Fasudil (CAS number: 105628-07-7), Y39983 (CAS number: 203911-26-6), Wf-536 (CAS Number: 539857-64-2), SLx-2119 (CAS Number: 911417-87-3), Azabenzimidazole-aminofurazans (CAS Number: 850664-21-0), DE-104, H-1152P (CAS number: 872543-07-6), a Rho kinase α inhibitor (a ROKα inhibitor), XD-4000, HMN-1152, 4-(1-anunoalkyl)-N-(4-pyridyl)cyclohexane-carboxamides (4-(1-aminoalkyl)-N-(4-pyridyl)cyclohexane-carboxamides), Rhostatin (Rhostain), BA-210, BA-207, Ki-23095, and VAS-012. Among these, Y-27632 is preferable.

The concentration of the ROCK signaling inhibitor contained in the culture medium is generally 1 µM to 20 µM and preferably 5 µM to 15 µM.

### (4) TGF-β signaling inhibitor

The downregulation of TGF-β signaling makes it possible to inhibit differentiation. Examples of the TGF-β signaling inhibitor include a TGF-β inhibitor associated with Smad2/3 phosphorylation and a BMP inhibitor associated with Smad1/5/9 phosphorylation.

Examples of the TGF-β inhibitor include A83-01 (CAS number: 909910-43-6), SB-431542 (CAS number: 301836-41-9), SB-505124 (CAS number: 694433-59-5), SB-525334 (CAS number: 356559-20-1), LY364947 (CAS number: 396129-53-6), SD-208 (CAS number: 627536-09-8), and SJN2511 (CAS number: 446859-33-2). Among these, A83-01 is preferable.

The concentration of the TGF-β inhibitor contained in the culture medium is generally 0.05 µM to 50 µM, preferably 0.5 µM to 30 µM, and more preferably 1 µM to 15 µM, and it is preferably 4 µM or more and 6 µM or less.

Examples of the BMP inhibitor include Noggin, Differential screening-selected gene Aberrative in Neuroblastoma (DAN), and a DAN-like protein. Among these, Noggin is preferable.

The concentration of the BMP inhibitor contained in the culture medium is generally 10 ng/mL to 100 ng/mL, preferably 15 ng/mL to 50 ng/mL, and more preferably 20 ng/mL to 30 ng/mL.

### (5) IL-6 family cytokine

An IL-6 family cytokine can enhance the proliferation of human hepatic progenitor cells. Examples of the IL-6 family cytokine include interleukin-6 (IL-6), interleukin-11 (IL-11), oncostatin M (OSM), leukemia inhibitory factor (LIF), cardiotrophin-1 (CT-1), and ciliary neurotrophic factor (CNTF). Among these, IL-6 is preferable.

The concentration of the IL-6 family cytokine contained in the culture medium is generally 10 ng/mL to 1.0 µg/mL, preferably 50 ng/mL to 500 ng/mL, more preferably 80 ng/mL to 200 ng/mL, and still more preferably 90 ng/mL to 110 ng/mL.

### (6) Retinoic acid

In the case where the culture medium contains retinoic acid, bile canaliculi can be increased, and cell aligning properties can be increased.

The concentration of the retinoic acid contained in the culture medium is generally 1 µM to 30 µM.

### (7) Nicotinamide

In the case where the culture medium contains nicotinamide, cell proliferation ability can be improved.

The concentration of the nicotinamide contained in the culture medium is generally 5 mM or less.

### (8) Forskolin

In the case where the culture medium further contains forskolin, the intracellular concentration of AMP can be increased, which can resultantly reactivate cellular receptors.

The concentration of the forskolin contained in the culture medium is generally 0.1 µM to 100 µM, preferably 1 µM to 50 µM, and more preferably 5 µM to 15 µM.

### (9) DAPT, DMSO, and Dex

In the case where the culture medium contains DAPT, DMSO, or Dex, the maturation into human hepatocyte-like cells can be enhanced.

### (10) Other components

In addition to the above components, the culture medium can further contain gastrin, a neurobiological supplement, N-acetylcysteine, and the like.

Examples of the neurobiological supplement include supplements containing insulin, such as a B27 supplement (Thermo Fisher Scientific, Inc.) and an N2 supplement (Thermo Fisher Scientific, Inc.).

The culture medium can be prepared by adding each component described above to a basal medium. Examples of the basal medium include Dulbecco's Modified Eagle's Medium (DMEM), Basal Medium (MEM), Knockout-DMEM (KO-DMEM), Glasgow Basal Medium (G-MEM), Eagle's Basal Medium (BME), DMEM/Ham's F12, Advanced DMEM/Ham's F12 (Advanced DMEM/F12), Iscove's Modified Dulbecco's Medium, Ham's F-10, Ham's F-12, 199 Medium, RPMI1640 Medium, and a human plasma mimetic medium.

### <Step B>

In step B, the culture formed in step A is dispersed to form a dispersion.

The culture formed in step A is a culture embedded in an extracellular matrix. In step B, the extracellular matrix is disrupted, and the culture is extracted. Examples of the method for disrupting the extracellular matrix include a method for physically disrupting the extracellular matrix and a method for chemically disrupting the extracellular matrix using an enzyme. Among these, the method for physically disrupting the extracellular matrix is preferable since damage to cells is small.

Next, the culture extracted from the extracellular matrix is dispersed to form a dispersion. Dispersion refers to separating cells into a cell population of 100 or less, preferably a cell population of 50 or less, and more preferably single cells, by a dispersion treatment such as an enzyme treatment or a physical treatment. Examples of the dispersion treatment include a mechanical dispersion treatment, a cell dispersion liquid treatment, and a treatment of adding a cell protecting agent. These treatments can be combined as appropriate. Among these, a cell dispersion liquid treatment is preferable.

Examples of the cell dispersion liquid used in the cell dispersion liquid treatment include a solution containing any of enzymes such as trypsin, collagenase, hyaluronidase, elastase, pronase, DNase, and papain; and a chelating agent such as ethylenediaminetetraacetic acid. Examples of the commercially available cell dispersion liquid include TrypLE Select and TrypLE Express manufactured by Thermo Fisher Scientific, Inc. Examples of the mechanical dispersion treatment include a pipetting treatment and a scraping operation with a scraper.

Before the dispersion treatment, a treatment with a cell protecting agent can be carried out to prevent cell death. Examples of the cell protecting agent include fibroblast growth factor (hereinafter, also referred to as "FGF"), heparin, a ROCK inhibitor, an insulin-like growth factor (hereinafter, also referred to as "1GF"), serum, and a serum substitute.

### <Step C>

In step C, the dispersion formed in step B is subjected to suspension culture in a culture medium, in which 0% to 10% by volume of an extracellular matrix is suspended with respect to a total volume of the culture medium, to form the sac-like material having a human hepatocyte-like cell.

Examples of the extracellular matrix in step C include the same extracellular matrices as those of exemplary examples in step 1.

The proportion of the extracellular matrix contained in the culture medium in step C is 0% to 10% by volume with respect to the total volume of the culture medium, where it is preferably 0.001% to 5% by volume, more preferably 0.1% to 4% by volume, and still more preferably 1 % to 3% by volume.

Examples of the method for suspending an extracellular matrix include a method in which a culture medium is mixed with an extracellular matrix precursor, and the extracellular matrix precursor is gelated to form an extracellular matrix. Examples of the method of mixing an extracellular matrix precursor include a pipetting method on an ice bath. Mixing means a state in which no extracellular matrix is visually observed in the culture medium.

Examples of the culture medium in step C include the culture media of exemplary examples in step A.

As the culture conditions in step C, conditions generally employed in the culturing of animal cells can be used. Examples thereof include atmosphere conditions in which the temperature is 30°C or higher and 40°C or lower and preferably 37°C, and the CO2 concentration is 5% by volume.

The culture time can be appropriately adjusted according to the number of cells, the state of the cells, and the like. It is generally one day or more and one week or less from the start of culture.

In the case where the sac-like material having a membrane of a human hepatocyte-like cell produced according to the production method according to the present embodiment is used as the present evaluation model for the evaluation method according to the present embodiment, since the culture medium after the culturing in step C contains the present evaluation model and 0% to 10% by volume of the suspended extracellular matrix, this culture medium can be used as the sac-like-material-containing solution in step 1 in the evaluation method according to the present embodiment.

### [Examples]

The present invention will be described with reference to experimental examples; however, the present invention is not limited to the experimental examples below.

### [Experimental Example 1]

### (Preparation of culture of human liver cell)

A human primary frozen floating hepatocyte (manufactured by BIOPRRIDIC International, HEP187-S) was thawed in a water bath at 37°C and suspended in a 50-mL tube to which a serum-free medium (a culture medium obtained by adding HEPES, GLUTAMAX, and PENICILIN/STEREPTOMYCIN to Advanced DMEM/F12) had been added, followed by centrifugation. After the centrifugation, the supernatant was removed, followed by suspension in a serum-free medium to prepare a human liver cell suspension. The 20,000 human liver cells from this suspension were mixed with 50 µL of Matrigel (manufactured by BD Biosciences) seeded in a 24-well tissue culture plate, and incubated at 37°C for 10 minutes until the Matrigel was completely polymerized. After the polymerization, 500 µL of the culture medium shown in Table 1 below was overlaid and cultured at 37°C in the presence of 5% by volume of CO₂ to obtain a culture of human liver cells. In the obtained culture, an inner cavity due to the membrane of the human hepatocyte-like cell was formed. The obtained culture was subcultured by repeating the same operation.

**[Table 1]**

| | Component | Concentration |
|---|---|---|
| Culture medium | R-Spondin 1 | 10% (v/v) |
| | Wnt3a | 20% (v/v) |
| | Noggin | 25 ng/mL |
| | EGF | 50 ng/mL |
| | FGF10 | 100 ng/mL |
| | HGF | 25 ng/mL |
| | Y-27632 | 10 µM |
| | Forskolin | 10 µM |
| | A83-01 | 5 µM |
| | IL-6 | 100 ng/mL |

### [Experimental Example 2]

### (Production of sac-like material (present evaluation model))

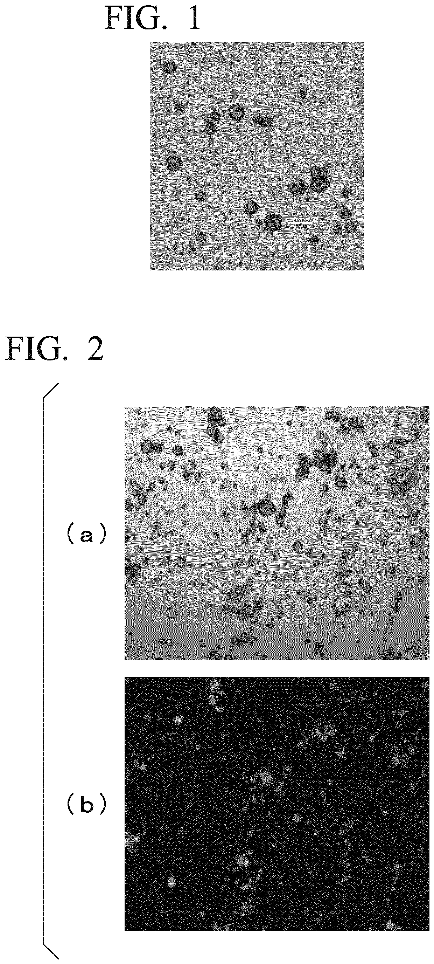

The culture of human liver cells after the subculturing in Experimental Example 1, the culture medium in Table 1, and 2% by volume of Matrigel with respect to the culture medium were added to a 6-well culture plate (manufactured by Sumitomo Bakelite Co., Ltd., MS-8006R), followed by the suspension culture at 37°C for 3 days in the presence of 5% by volume of CO₂ to form a sac-like material. FIG. 1 shows a bright-field image of the present evaluation model obtained by imaging the sac-like material-like matter with an optical microscope (magnification: 400 times (manufactured by KEYENCE CORPORATION, BZ-X700)).

### [Experimental Example 3]

### (Rho123 excretion of present evaluation model)

In Experimental Example 2, Rho123 (10 µM), which is a substrate for P-glycoprotein (P-gp), was added to the culture solution after the suspension culture. The incorporation of the Rho123 into the inner cavity of the present evaluation model was carried out at 37°C for 30 min in the presence of 5% by volume of CO₂. A culture solution containing the present evaluation model was placed in a low-adsorption 15 mL tube by pipetting, followed by centrifugation. After the centrifugation, the supernatant was removed, washing was carried out with PBS, and then it was confirmed that Rho123 was excreted into the inner cavity of the present evaluation model under an optical microscope (magnification: 400 times (manufactured by KEYENCE CORPORATION, BZ-X700)). A bright-field image ((a) of FIG. 2) and a fluorescence image ((b) of FIG. 2) are shown in FIG. 2.

As shown in FIG. 2, it was confirmed that Rho123 was excreted into the inner cavity of the present evaluation model.

### [Experimental Example 4]

### (Quantification of Rho 123 incorporated into inner cavity of present evaluation model)

The present evaluation model of Experimental Example 3, into which Rho123 had been incorporated, was mixed with methanol to kill the human hepatocyte-like cells. After mixing, centrifugation was carried out to extract the supernatant. The supernatant was subjected to analysis by a liquid chromatography-mass spectrometer (LC/MS) (manufactured by SHIMADZU CORPORATION, LCMSTM-8040), and from the concentration and amount of the Rho123 incorporated into the inner cavity and the concentration of the Rho123 in the culture solution, the proportion of the present evaluation model transported into the inner cavity was calculated. The LC/MS analysis conditions are shown below, and the obtained results are shown in Table 3.

### (LC conditions)

Column: Xbridge C18 3.5 µm, 2.1 mm × 50 mm
Column temperature: 40°C
Internal standard substance: Tolbutamine
Elution solvent: A solution (a 0.1% by mass formic acid solution), B solution (acetonitrile)
Gradient conditions: see Table 2
Runtime: 7 minutes
Flow rate: 0.4 mL/min
Injection volume: 3 µL

**[Table 2]**

| | | | | | | |
|---|---|---|---|---|---|---|
| Time (minutes) | 0 | 1 | 3 | 4.5 | 4.51 | 7 |
| A (% by volume) | 80 | 80 | 5 | 5 | 80 | STOP |
| B (% by volume) | 20 | 20 | 95 | 95 | 20 | |

### (MS conditions)

Mode: Positive (*the standard substance is in a negative mode)

**[Table 3]**

| | |
|---|---|
| Concentration of Rho123 in present evaluation model | 615.4 nM |
| Amount of Rho123 in present evaluation model | 0.123 nmol |
| Concentration of Rho123 in culture solution | 10 µM |
| Proportion transported into present evaluation model | 1.23% |

As shown in Table 3, the amount of the Rho123 incorporated into the inner cavity of the present evaluation model could be quantified, and the proportion of Rho123 in the culture solution, where the Rho123 had been transported into the inner cavity of the present evaluation model, could be calculated.

### [Experimental Example 5]

Rho123 (10 µM) was added to the culture solution containing the culture embedded in Matrigel after the culturing prepared in Experimental Example 1, and the incorporation of the Rho123 into the inner cavity of the culture was carried out at 37°C for 30 min in the presence of 5% by volume of CO2. The incorporation of Rho 123 into the inner cavity of the culture was confirmed with an optical microscope (magnification: 400 times (manufactured by KEYENCE CORPORATION, BZ-X700)). A bright-field image ((a) of FIG. 3) and a fluorescence image ((b) of FIG. 3) are shown in FIG. 3.

The polymerized Matrigel was degraded by pipetting and enzymatic treatment (Corning (registered trade name) Cell Recovery Solution) in order to measure the amount of the incorporated Rho123. However, the results were such that the Rho 123 incorporated from the liver organoids leaked out in association with the degradation of Matrigel, and thus it was not possible to measure the amount of the incorporated Rho123.

### [Experimental Example 6]

### (CDFDA excretion of present evaluation model)

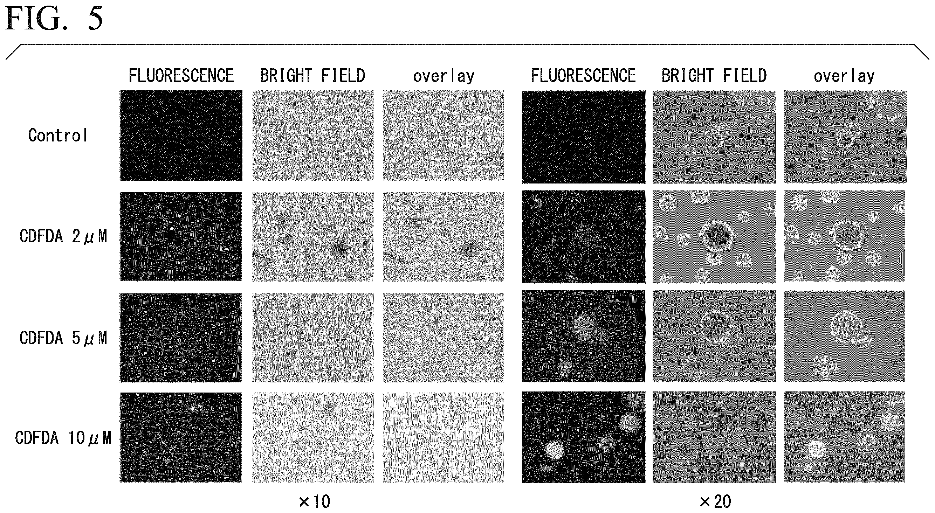

In Experimental Example 2, 2 µM, 5 µM, or 10 µM of carboxydichlorofluorescein diacetate (CDFDA), which is a substrate of MRP2, was added to the culture solution after the suspension culture. The incorporation of the CDFDA into the inner cavity of the present evaluation model was carried out at 37°C for 30 min in the presence of 5% by volume of CO₂. A culture solution containing the present evaluation model was placed in a low-adsorption 15 mL tube by pipetting, followed by centrifugation. After the centrifugation, the supernatant was removed, washing was carried out with PBS, and then it was confirmed that CDFDA was excreted into the inner cavity of the present evaluation model under an optical microscope (magnification: 400 times (manufactured by KEYENCE CORPORATION, BZ-X700)). The results are shown in FIG. 5.

### [Industrial Applicability]

According to the method of the present embodiment, it is possible to provide a method for evaluating the excretion of a substance of interest by a human hepatocyte-like cell using an evaluation model for evaluating metabolism or excretion by a human hepatocyte-like cell.

## Claims

1. A method for evaluating excretion of a substance of interest by a human hepatocyte-like cell, the method comprising:
preparing a sac-like-material-containing solution that contains a sac-like material which is produced in vitro and has a membrane of a human hepatocyte-like cell and 0% to 10% by volume of a suspended extracellular matrix;
placing the substance of interest in the sac-like-material-containing solution to bring the substance of interest into contact with the sac-like material; and
extracting the sac-like material from the sac-like-material-containing solution and measuring a concentration of the substance of interest or a metabolite thereof excreted into an inner cavity of the sac-like material.

2. The method according to Claim 1,
wherein the human hepatocyte-like cell is a cell capable of being subjected to organoid culture.

3. The method according to Claim 1 of 2,
wherein the human hepatocyte-like cell has at least one transporter selected from the group consisting of BSEP, MRP2, P-gp, MATE, and BCRP.

4. The method according to any one of Claims 1 to 3,
wherein in step 3, the concentration of the substance of interest or the metabolite thereof excreted into the inner cavity of the sac-like material is measured after degrading the sac-like material.

5. The method according to any one of Claims 1 to 4,
wherein in step 3, the concentration of the substance of interest of the metabolite thereof excreted into the inner cavity of the sac-like material is measured by liquid chromatography-mass spectrometry.

6. The method according to any one of Claims 1 to 5,
wherein the inner cavity of the sac-like material contains bile.

7. The method according to any one of Claims 1 to 6,
wherein the membrane of the human hepatocyte-like cell is a single layer membrane.

8. A method for producing a sac-like material having a membrane of a human hepatocyte-like cell, the method comprising:
embedding and culturing a human hepatic progenitor cell or a human liver cell in an extracellular matrix to form a culture;
dispersing the culture to form a dispersion; and
subjecting the dispersion to suspension culture in a culture medium, in which 0% to 10% by volume of an extracellular matrix is suspended with respect to a total volume of the culture medium, to form the sac-like material.
